# EUROPÄISCHE PATENTANMELDUNG

(11) **EP 3 777 968 A1**
(43) Veröffentlichungstag der Anmeldung: **17.02.2021**
(21) Anmeldenummer: 19191170.0
(22) Anmeldetag: 12.08.2019
(51) Int. Cl.: A61N 1/39, A61N 1/05

(54) **IMPLANTIERBARES DEFIBRILLATIONSSYSTEM**

(71) Anmelder: BIOTRONIK SE & Co. KG, 12359 Berlin (DE)
(72) Erfinder: DÖRR, Thomas, 12437 Berlin (DE); KOLBERG, Gernot, 12161 Berlin (DE)
(74) Vertreter: Galander, Marcus

(57) **Zusammenfassung**

Ein Defibrillationsgenerator (1) für ein implantierbares Defibrillationssystem umfasst ein Gehäuse (10), einen Anschlussblock (11) zum Anschließen einer Elektrodenleitungsbaugruppe (4), die nicht-transvenös in einem Patienten zu implantieren ist, und eine Steuereinrichtung (12) zum Steuern des Betriebs des Defibrillationsgenerators (1). Der Anschlussblock (11) weist zwei Anschlüsse (110, 112) auf, an die jeweils eine zumindest einen Defibrillationselektrodenpol (202) aufweisende Elektrodenleitung (2, 2A, 2B) der Elektrodenleitungsbaugruppe (4) anschließbar ist, wobei die Steuereinrichtung (12) zum Einnehmen eines ersten Betriebszustands, wenn nur an einen der zwei Anschlüsse (110, 112) eine Elektrodenleitung (2, 2A, 2B) angeschlossen ist, oder eines zweiten Betriebszustands, wenn an die zwei Anschlüsse (110, 112) jeweils eine Elektrodenleitung (2, 2A, 2B) angeschlossen ist, konfigurierbar ist.

## Beschreibung

Die vorliegende Erfindung betrifft einen Defibrillationsgenerator nach dem Oberbegriff des Anspruchs 1, ein implantierbares Defibrillationssystem und ein Verfahren zur Konfiguration eines implantierbaren Defibrillationssystems.

Ein solcher Defibrillationsgenerator umfasst ein Gehäuse, einen Anschlussblock zum Anschließen einer Elektrodenleitungsbaugruppe, die nicht-transvenös in einem Patienten zu implantieren ist, und eine Steuereinrichtung zum Steuern des Betriebs des Defibrillationsgenerators.

Ein solches Defibrillationssystem, auch bezeichnet als implantierbarer Cardioverter-Defibrillator (kurz: ICD), dient zur Erkennung und Behandlung von möglicherweise lebensbedrohlichen Herzrhythmusstörungen (Kammertachykardie, Bradykardie, Kammerflimmern). Ein solches Defibrillationssystem wird in einem Patienten implantiert derart, dass eine oder mehrere Elektrodenleitungen ausgehend von einem Defibrillationsgenerator sich hin zum menschlichen Herzen erstrecken, um dort zum einen Signale zum Zwecke der Erkennung von Herzrhythmusstörungen aufzunehmen und zum anderen Stimulationsenergie abzugeben, insbesondere um einen elektrischen Schock (Defibrillation) zu bewirken. Sowohl die Elektrodenleitungen als auch der Defibrillationsgenerator werden hierbei fest implantiert und verbleiben über einen längeren Zeitraum, üblicherweise mehrere Jahre, im Patienten.

Bei üblichen Defibrillationssystemen wird der Defibrillationsgenerator subkutan implantiert, und Elektrodenleitungen werden über einen Venenzugang transvenös direkt in das Herz hinein verlegt. Obwohl sich solche Defibrillationssysteme in der Praxis bewährt haben, kann wünschenswert sein, andere Defibrillationssysteme zur Verfügung zu stellen, die einfacher zu implantieren, gegebenenfalls durch Hinzufügen weiterer Elektrodenleitungen nachrüstbar und auch einfacher wieder zu entfernen sind, insbesondere unter Vermeidung eines direkten, transvenösen Zugangs in das Herz hinein.

Aufgabe der vorliegenden Erfindung ist es, einen Defibrillationsgenerator, ein implantierbares Defibrillationssystem und ein Verfahren zur Konfiguration eines implantierbaren Defibrillationssystems zur Verfügung zu stellen, die ein einfaches Implantieren bei variabler Verwendbarkeit und gegebenenfalls Nachrüstbarkeit mit zusätzlichen Elektrodenleitungen ermöglichen.

Diese Aufgabe wird durch einen Gegenstand mit den Merkmalen des Anspruchs 1 gelöst.

Demnach weist der Anschlussblock zwei Anschlüsse auf, an die jeweils eine zumindest einen Defibrillationselektrodenpol aufweisende Elektrodenleitung der Elektrodenleitungsbaugruppe anschließbar ist, wobei die Steuereinrichtung zum Einnehmen eines ersten Betriebszustands, wenn nur an einen der zwei Anschlüsse eine Elektrodenleitung angeschlossen ist, oder eines zweiten Betriebszustands, wenn an die zwei Anschlüsse jeweils eine Elektrodenleitung angeschlossen ist, konfigurierbar ist, um in dem ersten Betriebszustand eine Defibrillationsfunktion über die an den einen der Anschlüsse angeschlossene Elektrodenleitung und in dem zweiten Betriebszustand eine Defibrillationsfunktion über die an die zwei Anschlüsse angeschlossenen Elektrodenleitungen bereitzustellen.

Der Defibrillationsgenerator weist einen Anschlussblock auf, der zwei (oder mehr) Anschlüsse aufweist. Der Defibrillationsgenerator kann hierbei wahlweise mit einer oder mit mehreren Elektrodenleitungen einer nicht-transvenös in einem Patienten zu implantierenden Elektrodenleitungsbaugruppe bestückt werden, sodass der Defibrillationsgenerator mit lediglich einer angeschlossenen Elektrodenleitung oder mit zwei angeschlossenen Elektrodenleitungen oder gegebenenfalls auch mit mehr als zwei angeschlossenen Elektrodenleitungen betrieben werden kann.

Die Elektrodenleitungen der Elektrodenleitungsbaugruppe sind nicht-transvenös in einem Patienten zu implantieren. Hierunter ist zu verstehen, dass die Elektrodenleitungen der Elektrodenleitungsbaugruppe außerhalb des Herzens des Patienten zu verlegen sind, beispielsweise subkutan oder submuskulär. Die Elektrodenleitungen der nicht-transvenös zu implantierenden Elektrodenleitungsbaugruppe erstrecken sich somit nicht über einen venösen Zugang in das Herz des Patienten hinein, sondern verlaufen außerhalb des Herzens.

Nicht ausgeschlossen ist, dass über die Elektrodenleitungen der nicht-transvenös zu implantierenden Elektrodenleitungsbaugruppe hinaus weitere Elektrodenleitungen vorhanden sind, die gegebenenfalls transvenös im Herzen zu implantieren sind. Solche zusätzlichen Elektrodenleitungen können, wie nachfolgend noch erläutert werden soll, beispielsweise für eine intrakardiale Wahrnehmung und/oder Stimulation dienen.

Der Defibrillationsgenerator soll mit lediglich einer angeschlossenen Elektrodenleitung der Elektrodenleitungsbaugruppe oder mit mehr als einer angeschlossenen Elektrodenleitung der Elektrodenleitungsbaugruppe betrieben werden können. Jeweils ist der Defibrillationsgenerator hierbei zum Bereitstellen einer Defibrillationsfunktion ausgestaltet, sodass über eine angeschlossene Elektrodenleitungsbaugruppe elektrische Stimulationsenergie, beispielsweise mit einer maximalen Energie von 45 J oder darüber, abgegeben werden kann, um bei Detektion einer Herzrhythmusstörung eine Defibrillation zu bewirken.

Die Steuereinrichtung des Defibrillationsgenerators ist konfigurierbar, um einen ersten Betriebszustand oder einen zweiten Betriebszustand einzunehmen. Der erste Betriebszustand ist hierbei dazu ausgestaltet, eine Defibrillationsfunktion zur Verfügung zu stellen, wenn nur an einen der zwei Anschlüsse des Anschlussblocks des Defibrillationsgenerators eine Elektrodenleitung angeschlossen ist. Der zweite Betriebszustand ist demgegenüber dazu ausgestaltet, eine Defibrillationsfunktion zur Verfügung zu stellen, wenn an (zumindest) zwei Anschlüsse des Anschlussblocks Elektrodenleitungen angeschlossen sind.

Abhängig davon, ob der Defibrillationsgenerator mit einer Elektrodenleitung oder mit zwei Elektrodenleitungen bestückt ist, wird eine Defibrillationsfunktion somit über die eine angeschlossene Elektrodenleitung oder unter Verwendung von zwei angeschlossenen Elektrodenleitungen zur Verfügung gestellt. Zum Umschalten zwischen den unterschiedlichen Betriebszuständen kann die Steuereinrichtung beispielsweise eine Schalteinheit aufweisen, die beispielsweise durch eine elektronische Baugruppe verwirklicht oder durch eine Steuerungssoftware der Steuereinrichtung umgesetzt ist. Mittels der Schalteinheit werden in dem ersten Betriebszustand Signale lediglich einem Anschluss zugeleitet und gegebenenfalls von dem einen Anschluss empfangen. In dem zweiten Betriebszustand hingegen werden Signale an beide Anschlüsse geleitet und gegebenenfalls von beiden Anschlüssen zum Zwecke der Auswertung empfangen.

Dadurch, dass die Elektrodenleitungen der Elektrodenleitungsbaugruppe nicht-transvenös implantiert werden, ergibt sich eine einfache Implantationstechnik, beispielsweise unter Verwendung eines Tunnelierstabs. Ein Implantationsvorgang kann somit erheblich erleichtert sein, gegebenenfalls unter Vermeidung einer für die Implantation erforderlichen Vollnarkose und bei Reduzierung eines Operationsrisikos. Die einfache Implantation ermöglicht auch das einfache Nachrüsten durch Anschließen weiterer Elektrodenleitungen an einen bereits implantierten Defibrillationsgenerator, wobei zu diesem Zweck der Defibrillationsgenerator umkonfiguriert werden kann, um den Defibrillationsgenerator mit weiteren Elektrodenleitungen zu betreiben.

In einer Ausgestaltung weist der Anschlussblock einen dritten Anschluss zum Anschließen einer transvenös intrakardial zu implantierenden Elektrodenleitung auf. Über solch eine transvenös intrakardial zu implantierende Elektrodenleitung kann beispielsweise eine intrakardiale Stimulation und/oder Wahrnehmung von intrakardialen Signalen, beispielsweise atrialen Signalen und/oder ventrikulären Signalen, erfolgen. Eine solche zusätzliche dritte Elektrodenleitung ist, im Unterschied zu den Elektrodenleitungen der nicht-transvenös zu implantierenden Elektrodenleitungsbaugruppe, transvenös in das Herz hinein zu verlegen, sodass der Defibrillationsgenerator zusätzlich auch mit einer transvenösen Elektrodenleitung betrieben werden kann.

Zusätzlich oder alternativ kann der Anschlussblock einen vierten Anschluss zum Anschließen einer transvenös zu implantierenden Coronarsinus-Elektrodenleitung aufweisen. Eine solche Coronarsinus-Elektrodenleitung kann zur linksventrikulären Stimulation und/oder Wahrnehmung in das Herz hinein verlegt sein.

Jeweils kann die Steuereinrichtung konfigurierbar sein, um den Defibrillationsgenerator mit oder ohne eine an den dritten Anschluss und/oder den vierten Anschluss angeschlossene, zusätzliche, transvenöse Elektrodenleitung zu betreiben.

Sind zwei Elektrodenleitungen der Elektrodenleitungsbaugruppe, die jeweils einen Defibrillationselektrodenpol zum Abgeben von Stimulationspulsen zum Zwecke der Defibrillation aufweisen, an den Defibrillationsgenerator angeschlossen, kann eine Defibrillation unter Verwendung der einen Dipol ausbildenden Defibrillationselektrodenpole der Elektrodenleitungen vorgenommen werden. Ist lediglich eine Elektrodenleitung an einen der zwei Anschlüsse des Anschlussblocks angeschlossen, so kann die Elektrodenleitung gegebenenfalls zwei Defibrillationselektrodenpole aufweisen, die einen Dipol zum Abgeben von Stimulationsenergie zum Zwecke der Defibrillation ausbilden.

Sowohl bei zwei an den Defibrillationsgenerator angeschlossenen, jeweils einen Defibrillationselektrodenpol aufweisenden Elektrodenleitungen als auch bei einer einzigen an den Defibrillationsgenerator angeschlossenen, einen oder mehrere Defibrillationselektrodenpole aufweisenden Elektrodenleitung kann aber auch das Gehäuse des Defibrillationsgenerators als Gegenelektrode dienen, sodass ein Dipol zwischen einem Defibrillationselektrodenpol einer Elektrodenleitung und dem Gehäuse des Defibrillationsgenerators gebildet ist.

Der Defibrillationsgenerator kann hierbei konfiguriert werden, um Defibrillationsenergie über einen vorbestimmten Dipol (zwischen Defibrillationselektrodenpolen der Elektrodenleitungen oder zwischen einem Defibrillationselektrodenpol und dem Gehäuse des Defibrillationsgenerators) abzugeben, wobei die Konfiguration einmalig bei Inbetriebnahme oder wiederholt im Betrieb, zum Beispiel abhängig von einer effektiven Anregung, erfolgen kann.

Ein implantierbares Defibrillationssystem umfasst einen Defibrillationsgenerator der vorangehend beschriebenen Art und eine Elektrodenleitungsbaugruppe, die nicht-transvenös in einem Patienten zu implantieren ist. Die Elektrodenleitungsbaugruppe umfasst hierbei insbesondere eine erste Elektrodenleitung zum Anschließen an einen ersten Anschluss des Anschlussblocks des Defibrillationsgenerators und eine zweite Elektrodenleitung zum Anschließen an einen zweiten Anschluss des Anschlussblocks des Defibrillationsgenerators. Sowohl die erste Elektrodenleitung als auch die zweite Elektrodenleitung weisen zumindest einen Defibrillationselektrodenpol auf, sodass die Elektrodenleitungen gemeinsam oder gesondert voneinander zur Abgabe von Stimulationsenergie zum Zwecke der Defibrillation verwendet werden können.

Die Elektrodenleitungen können hierbei wahlweise an den Defibrillationsgenerator angeschlossen werden, wobei der Defibrillationsgenerator abhängig davon, welche Elektrodenleitungen an den Defibrillationsgenerator angeschlossen sind, unterschiedlich konfiguriert werden kann. Im Rahmen der Konfiguration kann auch festgelegt werden, über welchen Dipol (zwischen unterschiedlichen Defibrillationselektrodenpolen oder zwischen einem Defibrillationselektrodenpol und dem Gehäuse des Defibrillationsgenerators) im Betrieb gegebenenfalls Stimulationsenergie zum Zwecke der Defibrillation abgegeben werden soll.

In einer Ausgestaltung weisen die erste Elektrodenleitung und/oder die zweite Elektrodenleitung zumindest einen Wahrnehmungselektrodenpol zum Detektieren von ventrikulären und/oder atrialen Signalen auf. Über Elektrodenleitungen der nicht-transvenös zu implantierenden Elektrodenleitungsbaugruppe kann somit auch eine Wahrnehmung erfolgen, um Herzrhythmusstörungen zu detektieren und eine Stimulation zu steuern.

Solche Wahrnehmungselektrodenpole können zum Beispiel als Ringelektrode ausgebildet sein und erstrecken sich somit ringförmig um die zugeordnete Elektrodenleitung herum.

Ein Defibrillationselektrodenpol einer Elektrodenleitung kann demgegenüber als Wendel ausgebildet sein und erstreckt sich somit wendelförmig um die zugeordnete Elektrodenleitung herum. Aufgrund der wendelförmigen Ausgestaltung ist die Oberfläche des Defibrillationselektrodenpols vergrößert, sodass elektrische Schockenergie in effizienter Weise abgegeben und in das Herz eingeleitet werden kann, bei dennoch hinreichender Flexibilität an der Elektrodenleitung zur flexiblen, gegebenenfalls gekrümmten Verlegung im Patienten.

In einer Ausgestaltung ist ein atrialer Wahrnehmungselektrodenpol einer Elektrodenleitung der Elektrodenleitungsbaugruppe an einem distalen Ende der jeweiligen Elektrodenleitung angeordnet. Das distale Ende entspricht hierbei dem Ende der Elektrodenleitung, das einem an den Defibrillationsgenerator anschließbaren, proximalen Ende abgewandt ist. Der atriale Wahrnehmungselektrodenpol ist bei dieser Ausgestaltung somit distal von dem Defibrillationsgenerator platziert, wobei der atriale Wahrnehmungselektrodenpol unmittelbar am distalen Ende angeordnet sein kann und in diesem Fall beispielsweise die Spitze der Elektrodenleitung überdeckt. In implantiertem Zustand ist die Elektrodenleitung mit dem daran angeordneten atrialen Wahrnehmungselektrodenpol beispielsweise dem rechten Atrium des Herzens des Patienten angenähert, sodass über den atrialen Wahrnehmungselektrodenpol atriale Signale aufgenommen und dem Defibrillationsgenerator zugeleitet werden können.

Ein zur Aufnahme atrialer Signale dienender Wahrnehmungselektrodenpol kann zusätzlich oder alternativ aber auch proximal versetzt zum distalen Ende der Elektrodenleitung angeordnet sein, wobei der Wahrnehmungselektrodenpol aber distal vom proximalen Ende der Elektrodenleitung angeordnet ist.

In einer Ausgestaltung weist zumindest eine der Elektrodenleitungen der Elektrodenleitungsbaugruppe zwei Wahrnehmungselektrodenpole auf, die einen Dipol zum Detektieren atrialer Signale verwirklichen. Die zwei Wahrnehmungselektrodenpole können beispielsweise im Bereich des distalen Endes der Elektrodenleitung angeordnet sein. Möglich ist aber auch, dass die Wahrnehmungselektrodenpole weiter beabstandet voneinander an der Elektrodenleitung angeordnet sind. Zwischen den zwei Wahrnehmungselektrodenpolen können Signale aufgenommen werden, die ihren Ursprung in atrialer Aktivität des Herzens haben und somit ein Detektieren von atrialen Wahrnehmungssignalen ermöglichen.

In einer Ausgestaltung sind ein atrialer Wahrnehmungselektrodenpol an einer ersten Seite eines Defibrillationselektrodenpols und ein ventrikulärer Wahrnehmungselektrodenpol an einer zweiten Seite des Defibrillationselektrodenpols einer Elektrodenleitung angeordnet. Eine Wahrnehmung atrialer Signale und ventrikulärer Signale erfolgt somit und über unterschiedliche Wahrnehmungselektrodenpole an unterschiedlichen, axial zueinander versetzten Orten der Elektrodenleitung.

In einer Ausgestaltung weist das Defibrillationssystem eine dritte Elektrodenleitung zum Anschließen an einen dritten Anschluss des Anschlussblocks des Defibrillationsgenerators auf. Eine solche dritte Elektrodenleitung kann transvenös im Herzen zu implantieren sein und weist eine Elektrodenanordnung mit einem oder mehreren Elektrodenpolen auf, beispielsweise um intrakardial im rechten Ventrikel oder im rechten Atrium Signale aufzunehmen oder eine Stimulation zu bewirken. Die Steuereinrichtung des Defibrillationsgenerators kann hierbei derart konfigurierbar sein, dass Signale über die Elektrodenleitung zum Zwecke der Wahrnehmung empfangen und/oder Signale zum Zwecke der Stimulation an die dritte Elektrodenleitung abgegeben werden können.

In einer Ausgestaltung weist jede Elektrodenleitung der Elektrodenleitungsbaugruppe einen Stecker auf, der in einen zugeordneten Steckanschluss des Defibrillationsgenerators eingesteckt werden kann, um die Elektrodenleitung an den Defibrillationsgenerator anzuschließen. Über einen einzigen Stecker können hierbei beispielsweise sämtliche Elektrodenpole der Elektrodenleitung funktional mit dem Defibrillationsgenerator verbunden werden. Der Stecker kann beispielsweise Anschlusspole aufweisen, die bei steckendem Verbinden mit dem Steckanschluss des Defibrillationsgenerators mit zugeordneten Kontaktelementen des Steckanschlusses elektrisch kontaktieren, sodass elektrische Zuleitungen der Elektrodenleitung an den Defibrillationsgenerator angeschlossen werden.

Der Stecker kann beispielsweise zweipolig, vierpolig, sechspolig, achtpolig oder auch noch höherpolig sein.

Die Steuereinrichtung des Defibrillationsgenerators kann, in einer Ausgestaltung, einen ersten Empfangskanal zum Verarbeiten von über die Elektrodenleitungsbaugruppe empfangenen, ventrikulären Signalen und einen zweiten Empfangskanal zum Verarbeiten von über die Elektrodenleitungsbaugruppe empfangenen, atrialen Signalen ausweisen. Für das Aufnehmen und Verarbeiten atrialer Signale wird somit ein eigener Empfangskanal vorgesehen, in dem die atrialen Signale gesondert verarbeitet werden. Insbesondere kann der zweite Empfangskanal zum Verarbeiten der atrialen Signale eine andere Verstärkung und eine andere Filtercharakteristik als der erste Empfangskanal zum Verarbeiten der ventrikulären Signale aufweisen. Auf diese Weise können atriale Signale mit höherer Empfindlichkeit empfangen werden, beispielsweise mit einer Empfindlichkeit kleiner 1 mV, vorzugsweise kleiner 0,1 mV, weiter vorzugsweise kleiner 0,05 mV.

Atriale Signale können hierbei in besonderer Weise gefiltert werden, insbesondere zum Unterdrücken anderer, gegebenenfalls störender Signale, beispielsweise ventrikulärer Signale, zum Beispiel unter Verwendung eines zyklischen Zeitfensters, in dem störende Signale unterdrückt werden (sogenanntes "Blanking Window"). Atriale Signale können hierbei zudem verarbeitet werden, um Signalcharakteristiken zu analysieren, beispielsweise eine maximale (positive und/oder negative) Amplitude, einen Spitzen-zu-Spitzenwert, eine Pulsbreite oder einen (positiven und/oder negativen) Mittelwert.

Die Aufgabe wird auch gelöst durch ein Verfahren zur Konfiguration eines implantierbaren Defibrillationssystems, aufweisend: Bereitstellen eines Defibrillationsgenerators, der ein Gehäuse, einen Anschlussblock zum Anschließen einer Elektrodenleitungsbaugruppe und eine Steuereinrichtung zum Steuern des Betriebs des Defibrillationsgenerators aufweist; Bereitstellen einer zumindest eine Elektrodenleitung umfassenden Elektrodenleitungsbaugruppe, wobei die Elektrodenleitungsbaugruppe nicht-transvenös in einem Patienten zu implantieren ist; entweder Anschließen von einer zumindest einen Defibrillationselektrodenpol aufweisenden Elektrodenleitung der Elektrodenleitungsbaugruppe an einen von zwei Anschlüssen des Anschlussblocks oder Anschließen von zwei jeweils zumindest einen Defibrillationselektrodenpol aufweisenden Elektrodenleitungen der Elektrodenleitungsbaugruppe an die zwei Anschlüsse des Anschlussblocks; und Konfigurieren einer Schalteinheit der Steuereinrichtung zum Einnehmen eines ersten Betriebszustands, wenn nur an einen der zwei Anschlüsse eine Elektrodenleitung angeschlossen ist, oder eines zweiten Betriebszustands, wenn an die zwei Anschlüsse jeweils eine Elektrodenleitung angeschlossen ist, wobei die Steuereinrichtung in dem ersten Betriebszustand eine Defibrillationsfunktion über die an den einen der Anschlüsse angeschlossene Elektrodenleitung und in dem zweiten Betriebszustand eine Defibrillationsfunktion über die an die zwei Anschlüsse angeschlossenen Elektrodenleitungen bereitstellt.

Bezüglich des Verfahrens gelten die gleichen Vorteile und vorteilhaften Ausgestaltungen wie vorangehend für den Defibrillationsgenerator und das Defibrillationssystem beschrieben, sodass diesbezüglich vollumfänglich auf das vorangehend Ausgeführte verwiesen werden soll.

Die Konfiguration der Steuereinrichtung kann vor der Implantation des Defibrillationsgenerators und der Elektrodenleitungsbaugruppe, während der Implantation des Defibrillationsgenerators und der Elektrodenleitungsbaugruppe oder nach der Implantation des Defibrillationsgenerators und der Elektrodenleitungsbaugruppe erfolgen.

Der Defibrillationsgenerator kann hierbei zum Beispiel subkutan oder submuskulär in einem Patienten implantiert werden.

Eine oder mehrere Elektrodenleitungen der Elektrodenleitungsbaugruppen können beispielsweise unter Verwendung einer Tunneliertechnik subkutan oder submuskulär implantiert werden. Im Rahmen einer solchen Tunneliertechnik wird ein stumpfer Tunnelierstab, auf den ein sogenannter Introducer aufgeschoben ist, durch Gewebe des Patienten bewegt, um einen Implantierpfad zum Verlegen einer Elektrodenleitung vorzugeben. Der Tunnelierstab wird anschließend zurückgezogen, während der Introducer in seiner eingenommenen Position verbleibt und somit einen Tunnel ausbildet, durch den die Elektrodenleitung zum Zwecke der Implantation eingebracht werden kann.

Die Elektrodenleitung kann einen distalen Fixiermechanismus aufweisen, der aktiviert werden kann, wenn die Elektrodenleitung ihre bestimmungsgemäße Position erreicht hat.

Nach Einbringen der Elektrodenleitung kann der Introducer auf der Elektrode zurückgezogen werden, um den Introducer zu entfernen. Dies kann unter Aufspleißen des Introducers (dem sogenannten "Peelen") erfolgen.

Der der Erfindung zugrunde liegende Gedanke soll nachfolgend anhand der in den Figuren dargestellten Ausführungsbeispiele näher erläutert werden. Es zeigen:
- Fig. 1: eine Ansicht eines Defibrillationssystems mit einer nicht-transvenös implantierten, an einen Defibrillationsgenerator angeschlossenen Elektrodenleitung;
- Fig. 2: eine Ansicht eines Defibrillationssystems mit zwei nicht-transvenös implantierten, an einen Defibrillationsgenerator angeschlossenen Elektrodenleitungen;
- Fig. 3: eine Ansicht eines Defibrillationssystems mit zwei nicht-transvenös implantierten, an einen Defibrillationsgenerator angeschlossenen Elektrodenleitungen und einer zusätzlichen, transvenös implantierten Elektrodenleitung;
- Fig. 4: eine Ansicht eines Ausführungsbeispiels einer Elektrodenleitung;
- Fig. 5: eine Ansicht eines Defibrillationsgenerators des Defibrillationssystems; und
- Fig. 6: eine funktionale, schematische Ansicht einer Steuereinrichtung des Defibrillationsgenerators.

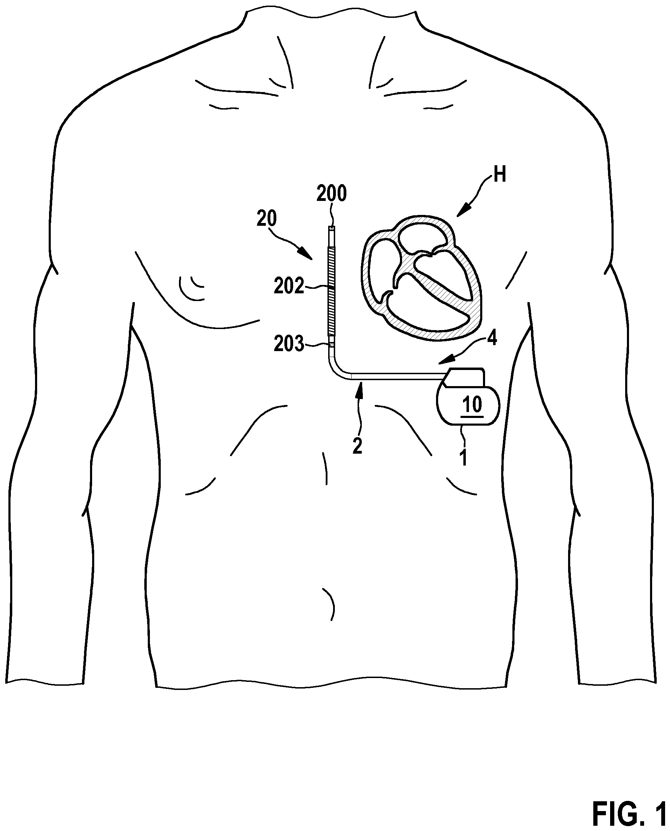

Bei einem in Fig. 1 dargestellten Ausführungsbeispiel weist ein implantierbares Defibrillationssystem einen Defibrillationsgenerator 1 auf, der beispielsweise subkutan oder unter dem Brustmuskel eines Patienten zu implantieren ist und an den eine Elektrodenleitung 2 (oder mehrere Elektrodenleitung, wie nachfolgend noch beschrieben werden soll) einer Elektrodenleitungsbaugruppe 4 angeschlossen sind. Die Elektrodenleitung 2 erstreckt sich hierbei in implantiertem Zustand von dem Defibrillationsgenerator 1 außerhalb des Herzens H des Patienten und ist somit nicht-transvenös, ohne direkten Zugang in das Herz hinein, implantiert.

Wie aus Fig. 1 ersichtlich ist, verläuft die Elektrodenleitung 2 somit außerhalb des Herzens H des Patienten. Eine im distalen Bereich an der Elektrodenleitung 2 angeordnete Elektrodenanordnung 20 ist dem Herzen H von außen angenähert, sodass über die Elektrodenanordnung 20 Signale des Herzens H aufgenommen und zudem elektrische Signale zum Zwecke der Stimulation an das Herz H abgegeben werden können.

Die Elektrodenanordnung 20 der Elektrodenleitung 2 ist bei dem Beispiel gemäß Fig. 1 in implantiertem Zustand insbesondere dem rechten Atrium RA und dem rechten Ventrikel RV des Herzens H angenähert. Über die Elektrodenanordnung 20 können beispielsweise sowohl atriale Signale aus dem rechten Atrium RA als auch ventrikuläre Signale aus dem rechten Ventrikel RV aufgenommen und zudem Stimulationsenergie zum Zwecke der Stimulation insbesondere zur Behandlung von Herzrhythmusstörungen abgegeben werden.

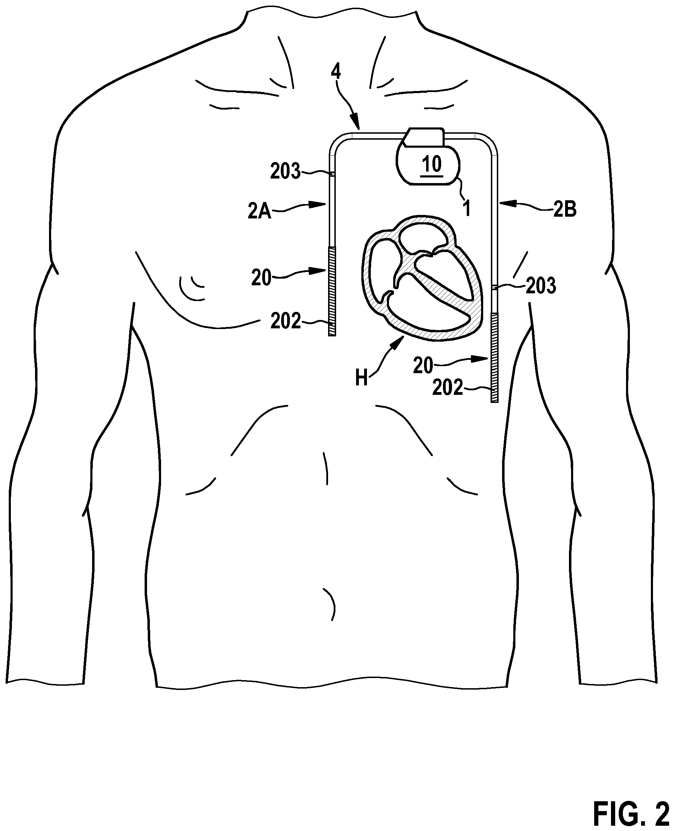

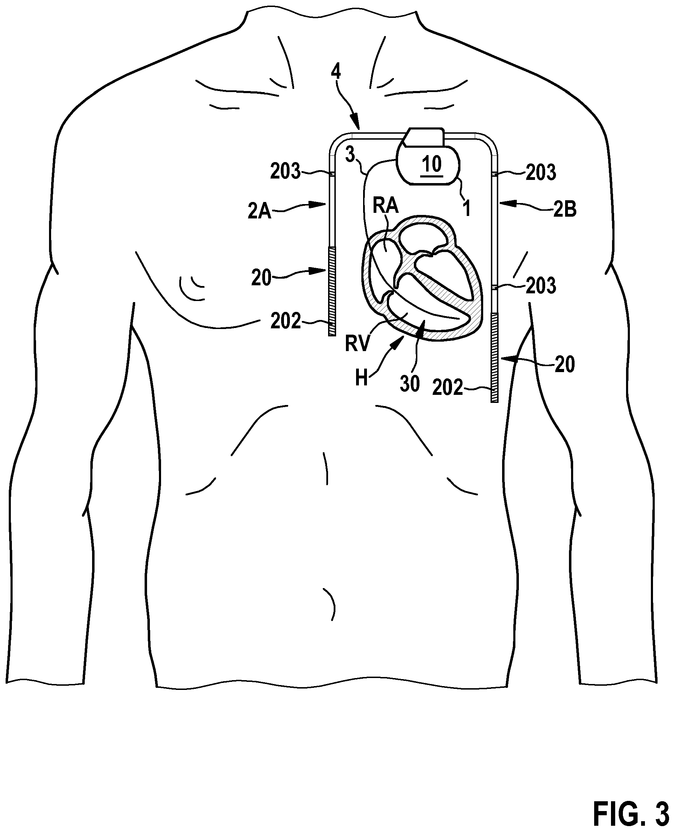

Bei dem in Fig. 1 dargestellten Ausführungsbeispiel ist eine (einzige) nicht-transvenöse Elektrodenleitung 2 an den Defibrillationsgenerator 1 angeschlossen. Bei in Fig. 2 und 3 dargestellten Ausführungsbeispielen sind demgegenüber jeweils zwei Elektrodenleitungen 2A, 2B einer nicht-transvenösen Elektrodenleitungsbaugruppe 4 an den Defibrillationsgenerator 1 angeschlossen, wobei die Elektrodenleitungen 2A, 2B jeweils nicht-transvenös und somit außerhalb des Herzens H des Patienten verlegt sind. Jede Elektrodenleitung 2A, 2B weist eine Elektrodenanordnung 20 mit Elektrodenpolen 202, 203 auf.

Bei dem in Fig. 3 dargestellten Ausführungsbeispiel ist, im Vergleich zum Ausführungsbeispiel gemäß Fig. 2, zusätzlich eine weitere Elektrodenleitung 3 an den Defibrillationsgenerator 1 angeschlossen, die transvenös implantiert ist und sich in das Herz H hinein erstreckt, insbesondere - bei dem dargestellten Ausführungsbeispiel - in den rechten Ventrikel RV.

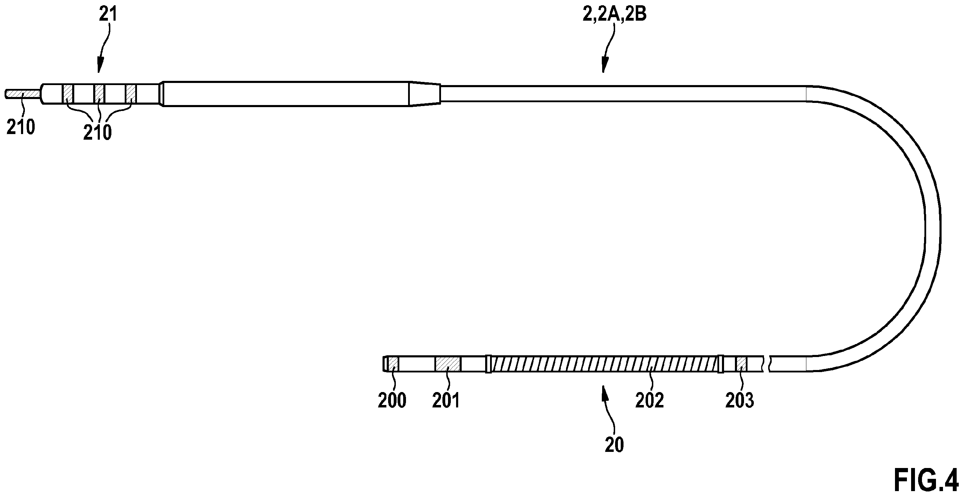

Fig. 4 zeigt ein Ausführungsbeispiel einer Elektrodenleitung 2, 2A, 2B, wie sie bei den Ausführungsbeispielen gemäß Fig. 1 bis 3 allein (Fig. 1) oder gemeinsam mit einer oder mehreren weiteren Elektrodenleitungen (Fig. 2 und 3) an einen Defibrillationsgenerator 1 angeschlossen werden kann. Die Elektrodenleitung 2, 2A, 2B weist bei dem dargestellten Ausführungsbeispiel eine Elektrodenanordnung 20 auf, die durch eine Mehrzahl von Elektrodenpolen 200-203 gebildet ist und im Bereich eines distalen Endes der Elektrodenleitung 2 angeordnet ist. An einem proximalen Ende weist die Elektrodenleitung 2, 2A, 2B einen Stecker 21 auf, an dem Anschlusspole 210 angeordnet sind und der steckend mit dem Defibrillationsgenerator 1 verbunden werden kann, um auf diese Weise die Elektrodenpole 200-203 an den Defibrillationsgenerator 1 anzuschließen, empfangene Signale dem Defibrillationsgenerator 1 zuzuleiten und Stimulationsenergie hin zu der Elektrodenanordnung 20 zu führen.

Die Elektrodenanordnung 20 weist eine Mehrzahl von Elektrodenpolen 200-203 auf. Einem jeden Elektrodenpol 200-203 kann hierbei ein Anschlusspol 210 zugeordnet sein, wobei in diesem Fall der jeweilige Elektrodenpol 200-203 über eine Zuleitung mit dem zugeordneten Anschlusspol 210 verbunden ist. Die Anzahl der Anschlusspole 210 entspricht in diesem Fall der Anzahl der Elektrodenpole 200-203. Alternativ können einzelne Elektrodenpole 200-203 auch gemeinsam an einen zugeordneten Anschlusspol 210 angeschlossen sein.

Bei dem dargestellten Ausführungsbeispiel weist der Stecker 21 vier Anschlusspole 210 auf und ist somit vierpolig ausgebildet. Alternativ kann der Stecker 21 auch zum Beispiel eine höhere Anzahl von Anschlusspolen 210 aufweisen und beispielsweise sechspolig oder achtpolig ausgebildet sein.

Die Elektrodenpole 200-203 sind unterschiedlich ausgestaltet und erfüllen unterschiedliche Funktionen.

Ein erster, als Defibrillationselektrodenpol bezeichneter Elektrodenpol 202 dient zur Abgabe von Stimulationsenergie und ist wendelförmig an der Elektrodenleitung 2, 2A, 2B angeordnet. Bei dem dargestellten Ausführungsbeispiel weist die Elektrodenleitung 2, 2A, 2B einen Defibrillationselektrodenpol 202 auf, dem Stimulationsenergie vom Defibrillationsgenerator 1 zugeführt werden kann, um Stimulationsenergie zum Zwecke der Defibrillation an das Herz H abzugeben und auf diese Weise detektierte Herzrhythmusstörungen zu behandeln.

Ein zweiter, als ventrikulärer Wahrnehmungselektrodenpol bezeichneter Elektrodenpol 203 dient zum Beispiel zum Detektieren von ventrikulären Signalen, also Signalen, die einen ventrikulären Ursprung haben und auf eine ventrikuläre Aktivität am Herzen H zurückgehen. Solche ventrikulären Signale können intrinsisch sein, also auf eine Eigenaktivität des Herzens zurückgehen. Solche ventrikuläre Signale können aber auch stimuliert sein.

Dritte, als atriale Wahrnehmungselektrodenpole bezeichnete Elektrodenpole 200, 201 sind bei dem in Fig. 4 dargestellten Ausführungsbeispiel im Bereich des distalen Endes der Elektrodenleitung 2 angeordnet. Die Wahrnehmungselektrodenpole 200, 201 dienen zum Beispiel zum Detektieren von atrialen Signalen und sind, bei implantierter Elektrodenleitung 2, 2A, 2B, in unmittelbarer räumlicher Nähe beispielsweise zum rechten Atrium RA des Herzens H angeordnet.

Während der Defibrillationselektrodenpol 202 durch eine Wendel ausgebildet ist, sind der ventrikuläre Wahrnehmungselektrodenpol 203 und die atrialen Wahrnehmungselektrodenpole 200, 201 beispielsweise als Ringelektroden ausgebildet, die sich ringförmig um die Elektrodenleitung 2 erstrecken.

Jeweils können die Elektrodenpole 200-203 nach außen hin freiliegen und somit mit umliegendem Gewebe des Patienten elektrisch kontaktieren.

Die atrialen Wahrnehmungselektrodenpole 200, 201 bilden bei dem dargestellten Ausführungsbeispiel gemeinsam einen Dipol aus, über den atriale Signale empfangen werden können.

Der Defibrillationsgenerator 1 ist, wie beispielhaft in Fig. 1-3 dargestellt, dazu ausgestaltet, dass eine oder mehrere Elektrodenleitungen 2, 2A, 2B an den Defibrillationsgenerator 1 angeschlossen werden können. Die Elektrodenleitungen 2, 2A, 2B können hierbei baugleich und zum Beispiel jeweils wie in Fig. 4 dargestellt ausgestaltet sein. Möglich ist aber auch, dass unterschiedlich ausgestaltete Elektrodenleitungen 2, 2A, 2B, beispielsweise Elektrodenleitungen 2, 2A, 2B mit unterschiedlicher Anzahl und Anordnung von Elektrodenpolen, an den Defibrillationsgenerator 1 angeschlossen werden können.

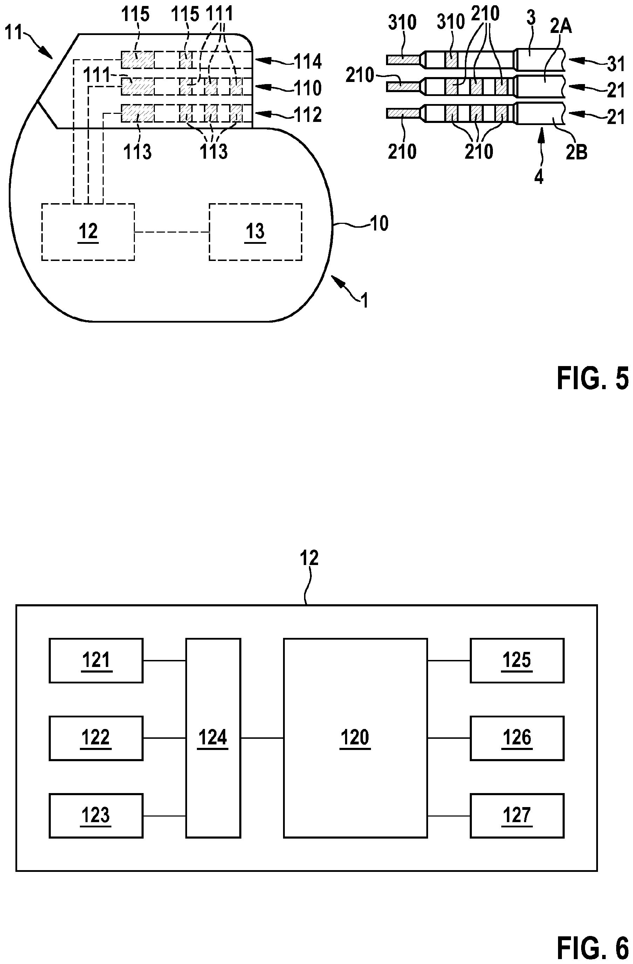

Wie in einem Ausführungsbeispiel in Fig. 5 dargestellt, weist der Defibrillationsgenerator 1 ein Gehäuse 10 und einen an dem Gehäuse 10 ausgebildeten Anschlussblock 11 auf. Der Anschlussblock 11 weist Anschlüsse 110, 112, 114 auf, an die Elektrodenleitungen 2, 2A, 2B, 3 angeschlossen werden können, wie dies beispielhaft in Fig. 5 dargestellt ist. Ein jeder Anschluss 110, 112, 114 bildet hierbei einen Steckanschluss aus, in den ein zugeordneter Stecker 21, 31 einer zugeordneten Elektrodenleitung 2, 2A, 2B, 3 eingesteckt werden kann derart, dass Anschlusspole 210, 310 der Stecker 21, 31 mit zugeordneten Kontaktelementen 111, 113, 115 der Anschlüsse 110, 112, 114 elektrisch kontaktieren und somit eine elektrische Verbindung zwischen den Elektrodenleitungen 2, 2A, 2B, 3 und dem Defibrillationsgenerator 1 herstellen.

Der Defibrillationsgenerator 1 weist eine Steuereinrichtung 12 auf, die funktional mit den Anschlüssen 110, 112, 114, insbesondere den Kontaktelementen 111, 113, 115 der Anschlüsse 110, 112, 114 verbunden ist, sodass Signale von den Anschlüssen 110, 112, 114 empfangen und Signale den Anschlüssen 110, 112, 114 zugeleitet werden können. Der Defibrillationsgenerator 1 weist zudem einen Energiespeicher 13 insbesondere in Form einer Batterie auf. Der Defibrillationsgenerator 1 kann in einem Patienten implantiert werden und soll über einen langen Zeitraum, beispielsweise mehrere Jahre, im Patienten verbleiben. Sämtliche Komponenten des Defibrillationsgenerators 1 sind über das Gehäuse 10 gekapselt und damit feuchtigkeitsdicht eingeschlossen.

Die Steuereinrichtung 12 ist zum Einnehmen unterschiedlicher Betriebszustände konfigurierbar. Abhängig davon, in welcher Anzahl und Kombination Elektrodenleitungen 2, 2A, 2B, 3 an den Defibrillationsgenerator 1 angeschlossen sind, kann die Steuereinrichtung 12 in unterschiedlichen Betriebszuständen betrieben werden, um jeweils eine Defibrillationsfunktion zur Behandlung von Herzrhythmusstörungen zur Verfügung zu stellen.

Wie schematisch in Fig. 6 dargestellt, weist die Steuereinrichtung 12 beispielsweise eine erste Anschlusseinheit 121 auf, die einem ersten Anschluss 110 zugeordnet ist, eine zweite Anschlusseinheit 122, die einem zweiten Anschluss 112 zugeordnet ist, und eine dritte Anschlusseinheit 123, die einem dritten Anschluss 114 zugeordnet ist, auf. Die Anschlusseinheiten 121-123 sind jeweils mit einer Schalteinheit 124 verbunden, über die die Anschlusseinheiten 121-123 in variabel konfigurierbarer Weise angesteuert werden können.

Ist beispielsweise, wie in Fig. 1 dargestellt, lediglich eine Elektrodenleitung 2 an den Anschluss 110 angeschlossen, so kann die Steuereinrichtung 12 konfiguriert werden, um lediglich über die Anschlusseinheit 121 Signale zu empfangen und abzugeben, sodass eine Defibrillationsfunktion über die an den Anschluss 110 angeschlossene Elektrodenleitung 2 bereitgestellt wird.

Sind demgegenüber zwei Elektrodenleitungen 2A, 2B, wie in Fig. 2 dargestellt, an die Anschlüsse 110, 112 angeschlossen, so werden Signale über die Anschlusseinheiten 121, 122 empfangen und abgegeben, sodass eine Defibrillationsfunktion unter Verwendung der Elektrodenleitungen 2A, 2B zur Verfügung gestellt werden kann.

Ist zusätzlich eine transvenös implantierte Elektrodenleitung 3 an den Anschluss 114 angeschlossen, so werden zusätzlich Signale auch über die Anschlusseinheit 123 empfangen und gegebenenfalls auch zur intrakardialen Stimulation abgeben.

Die Schalteinheit 124 kann durch eine elektronische Baugruppe verwirklicht sein. Denkbar ist auch, dass die Schalteinheit 124 durch eine Steuerungssoftware und somit softwaretechnisch (ohne einen Hardwareschalter) umgesetzt ist.

Die Schalteinheit 124 ist funktional mit einer Steuereinheit 120 der Steuereinrichtung 12 verbunden, die empfangene Signale zum Zwecke einer Diagnostik und Therapiesteuerung auswertet. Die Steuereinheit 120 ist mit einer ventrikulären Stimulationseinheit 125, einer atrialen Stimulationseinheit 126 und einer Kardioversions-/Defibrillationseinheit 127 verbunden, über die Signale für eine ventrikuläre und/oder atriale Stimulation und für eine Defibrillation generiert und angeschlossenen Elektrodenleitungen 2, 2A, 2B, 3 zugeleitet werden können.

Die Konfiguration der Steuereinrichtung 12 kann vor Implantation, bei Implantation oder auch nach einer Implantation erfolgen. Die Konfiguration erfolgt durch Programmierung, wobei durch die Konfiguration die Steuereinrichtung 12 einer angeschlossenen Kombination von Elektrodenleitungen 2, 2A, 2B, 3 angepasst wird, um entsprechend über eine angeschlossene Kombination von Elektrodenleitungen 2, 2A, 2B, 3 eine Defibrillationsfunktion zur Verfügung stellen zu können.

Gegebenenfalls kann die Steuereinrichtung 12 auch dazu ausgestaltet sein, selbsttätig zu erkennen, welche Art und Kombination von Elektrodenleitungen 2, 2A, 2B, 3 an den Defibrillationsgenerator 1 angeschlossen sind, um selbsttätig eine Konfiguration durchzuführen.

Die Abgabe von Defibrillationsenergie (Defibrillationsschocks) erfolgt über Defibrillationselektrodenpole 202 von angeschlossenen, nicht-transvenös implantierten Elektrodenleitungen 2, 2A, 2B.

Bei dem in Fig. 1 dargestellten Ausführungsbeispiel weist die angeschlossene Elektrodenleitung 2 hierbei einen einzigen Defibrillationselektrodenpol 202 auf, der gemeinsam mit dem Gehäuse 10 des Defibrillationsgenerators 1 einen Dipol ausbildet. Das Gehäuse 10 stellt somit eine Gegenelektrode für den Defibrillationselektrodenpol 202 der Elektrodenleitung 2 dar, sodass zwischen dem Defibrillationselektrodenpol 202 und dem Gehäuse 10 Stimulationsenergie in das Herz H injiziert werden kann.

Bei den in Fig. 2 und 3 dargestellten Ausführungsbeispielen weisen die an den Defibrillationsgenerator 1 angeschlossenen, nicht-transvenös implantierten Elektrodenleitungen 2A, 2B jeweils einen Defibrillationselektrodenpol auf. Stimulationsenergie kann zwischen den beiden Defibrillationselektrodenpolen 202 der Elektrodenleitungen 2A, 2B abgegeben werden. Alternativ kann auch das Gehäuse 10 des Defibrillationsgenerators 1 als Gegenelektrode mit einbezogen werden, beispielsweise indem Stimulationsenergie zwischen dem Defibrillationselektrodenpol 202 einer der Elektrodenleitungen 2A, 2B und der Kombination des Defibrillationselektrodenpols 202 der anderen Elektrodenleitung 2B, 2A und des Gehäuses 10 des Defibrillationsgenerators 1 eingespeist wird (der Defibrillationselektrodenpol 202 der anderen Elektrodenleitung 2B, 2A und das Gehäuse 10 des Defibrillationsgenerators 1 dienen insofern gemeinsam als Gegenelektrode).

Bei dem Beispiel gemäß Fig. 1 können über Wahrnehmungselektrodenpole 200, 203 atriale und/oder ventrikuläre Signale aufgenommen werden. Wahrnehmungssignale können hierbei zwischen einem jeden der Wahrnehmungselektrodenpole 200, 203 und dem Gehäuse 10 des Defibrillationsgenerators 1 als Gegenelektrode aufgenommen werden. Zudem können Signale zwischen den Wahrnehmungselektroden 200, 203, die gemeinsam einen Dipol ausbilden, aufgenommen werden. Aus den Wahrnehmungssignalen kann ein EKG abgeleitet werden, anhand dessen eine Diagnostik und Therapiesteuerung für eine eventuell vorzunehmende Defibrillation vorgenommen werden kann.

Bei dem in Fig. 2 dargestellten Beispiel weist jede Elektrodenleitung 2A, 2B einen Wahrnehmungselektrodenpol 203 auf. Wahrnehmungssignale können entsprechend zwischen den Wahrnehmungselektrodenpolen 203 der Elektrodenleitungen 2A, 2B aufgenommen werden (die gemeinsam einen Dipol ausbilden). Zusätzlich oder alternativ können Wahrnehmungssignale zwischen dem Wahrnehmungselektrodenpol 203 einer jeden Elektrodenleitung 2A, 2B und dem Gehäuse 10 des Defibrillationsgenerators 1 als Gegenelektrode aufgenommen werden. Wiederum kann aus den Wahrnehmungssignalen ein EKG abgeleitet werden, das zur Diagnostik und Therapiesteuerung ausgewertet werden kann.

Bei dem Beispiel gemäß Fig. 3 kann über die zusätzliche Elektrodenleitung 3 und die daran distal angeordnete Elektrodenanordnung 30 beispielsweise eine rechtsventrikuläre antibradykarde Stimulation und/oder eine antitachykarde Stimulation (ATP) erfolgen. Zusätzlich oder alternativ können über die Elektrodenanordnung 30 atriale und/oder ventrikuläre Signale zum Zwecke der Diagnostik und Therapiesteuerung aufgenommen werden.

Eine Elektrodenleitung 3 kann beispielsweise zu einem späteren Zeitpunkt nach einer Erstimplantation des Defibrillationssystems hinzugefügt werden, zum Beispiel wenn in dem Patienten wiederholt langsame ventrikuläre Tachykardien vorkommen oder die Wahrnehmung von Signalen zum Zwecke der Diagnostik und Therapiesteuerung verbessert werden soll, weil beispielsweise eine Wahrnehmung über die nicht-transvenösen Elektrodenleitungen 2A, 2B nicht hinreichend ist.

Zusätzlich oder alternativ kann beispielsweise eine transvenöse Coronarsinus-Elektrode an den Defibrillationsgenerator 1 angeschlossen werden, der hierzu einen zusätzlichen, vierten Anschluss aufweisen kann. Über eine solche Coronarsinus-Elektrode kann eine linksventrikuläre Stimulation und/oder Wahrnehmung erfolgen.

Elektrodenleitungen 2, 2A, 2B, 3 können durch eine geeignete Markierung, zum Beispiel einen Text, eine Farbmarkierung oder eine radiopaque Markierung, markiert sein. Ebenso können Anschlüsse 110, 112, 114 des Defibrillationsgenerators 1 markiert sein.

Weil eine aus den Elektrodenleitungen 2, 2A, 2B bestehende Elektrodenleitungsbaugruppe 4 nicht-transvenös implantiert wird, ergibt sich eine einfache Implantation des Defibrillationssystems. Insbesondere kann der Deflationsgenerator 1 subkutan oder submuskulär, zum Beispiel unterhalb des Schlüsselbeins eines Patienten, implantiert werden. Elektrodenleitungen 2, 2A, 2B können zum Beispiel unter Verwendung einer Tunneliertechnik beispielsweise parasternal implantiert werden und erstrecken sich somit außerhalb des Herzens H des Patienten.

Der der Erfindung zugrunde liegende Gedanke ist nicht auf die vorangehend beschriebenen Ausführungsbeispiele beschränkt, sondern lässt sich auch in anderer Weise verwirklichen.

Elektrodenleitungen können anders als bei den dargestellten Beispielen aufgebaut sein und können insbesondere andere Kombinationen und Anordnungen von Elektrodenpolen aufweisen.

An einen Defibrillationsgenerator können hierbei ein oder mehrere Elektrodenleitungen anzuschließen sein, wobei zumindest einige der Elektrodenleitungen nicht-transvenös zu implantieren sind.

### Liste der Bezugszeichen

- 1: Defibrillationsgenerator
- 10: Gehäuse
- 11: Anschlussblock
- 110: Steckanschluss
- 111: Kontaktelement
- 112: Steckanschluss
- 113: Kontaktelement
- 114: Steckanschluss
- 115: Kontaktelement
- 12: Steuereinrichtung
- 120: Steuereinheit
- 121-123: Anschlusseinheit
- 124: Schalteinheit
- 125: Ventrikuläre Stimulationseinheit
- 126: Atriale Stimulationseinheit
- 127: Kardioversions-/Defibrillationseinheit
- 13: Energiequelle (Batterie)
- 2, 2A, 2B: Elektrodenleitung
- 20: Elektrodenanordnung
- 200, 201, 203: Wahrnehmungselektrodenpol
- 202: Defibrillationselektrodenpol
- 21: Stecker
- 210: Anschlusspol
- 3: Elektrodenleitung
- 30: Elektrodenanordnung
- 31: Stecker
- 4: Elektrodenleitungsbaugruppe
- H: Herz
- RA: Rechtes Atrium
- RV: Rechter Ventrikel

## Patentansprüche

1. Defibrillationsgenerator (1) für ein implantierbares Defibrillationssystem, mit einem Gehäuse (10), einem Anschlussblock (11) zum Anschließen einer Elektrodenleitungsbaugruppe (4), die nicht-transvenös in einem Patienten zu implantieren ist, und einer Steuereinrichtung (12) zum Steuern des Betriebs des Defibrillationsgenerators (1), **dadurch gekennzeichnet, dass** der Anschlussblock (11) zwei Anschlüsse (110, 112) aufweist, an die jeweils eine zumindest einen Defibrillationselektrodenpol (202) aufweisende Elektrodenleitung (2, 2A, 2B) der Elektrodenleitungsbaugruppe (4) anschließbar ist, wobei die Steuereinrichtung (12) zum Einnehmen eines ersten Betriebszustands, wenn nur an einen der zwei Anschlüsse (110, 112) eine Elektrodenleitung (2, 2A, 2B) angeschlossen ist, oder eines zweiten Betriebszustands, wenn an die zwei Anschlüsse (110, 112) jeweils eine Elektrodenleitung (2, 2A, 2B) angeschlossen ist, konfigurierbar ist, um in dem ersten Betriebszustand eine Defibrillationsfunktion über die an den einen der Anschlüsse (110, 112) angeschlossene Elektrodenleitung (2, 2A, 2B) und in dem zweiten Betriebszustand eine Defibrillationsfunktion über die an die zwei Anschlüsse (10, 112) angeschlossenen Elektrodenleitungen (2, 2A, 2B) bereitzustellen.

2. Defibrillationsgenerator (1) nach Anspruch 1, **dadurch gekennzeichnet, dass** der Anschlussblock (11) einen dritten Anschluss (114) zum Anschließen einer transvenös intrakardial zu implantierenden Elektrodenleitung (3) aufweist.

3. Defibrillationsgenerator (1) nach Anspruch 1 oder 2, **dadurch gekennzeichnet dass** der Anschlussblock (11) einen vierten Anschluss zum Anschließen einer transvenös zu implantierenden Coronarsinus-Elektrodenleitung aufweist.

4. Defibrillationsgenerator (1) nach einem der Ansprüche 1 bis 3, **dadurch gekennzeichnet, dass** das Gehäuse (10) des Defibrillationsgenerators (1) eine Gegenelektrode zu zumindest einem Defibrillationselektrodenpol (202) einer Elektrodenleitung (2, 2A, 2b) der Elektrodenleitungsbaugruppe (4) zum Abgeben von Stimulationsenergie ausbildet.

5. Implantierbares Defibrillationssystem, mit einem Defibrillationsgenerator (1) nach einem der vorangehenden Ansprüche und einer Elektrodenleitungsbaugruppe (4), die nicht-transvenös in einem Patienten zu implantieren ist.

6. Implantierbares Defibrillationssystem nach Anspruch 5, **dadurch gekennzeichnet, dass** die Elektrodenleitungsbaugruppe (4) eine erste Elektrodenleitung (2A) zum Anschließen an einen ersten Anschluss (110) des Anschlussblocks (11) des Defibrillationsgenerators (1) und eine zweite Elektrodenleitung (2B) zum Anschließen an einen zweiten Anschluss (112) des Anschlussblocks (11) des Defibrillationsgenerators (1) aufweist, wobei die erste Elektrodenleitung (2A) und die zweite Elektrodenleitung (2B) jeweils zumindest einen Defibrillationselektrodenpol (202) aufweisen.

7. Implantierbares Defibrillationssystem nach Anspruch 6, **dadurch gekennzeichnet, dass** die erste Elektrodenleitung (2A) und/oder die zweite Elektrodenleitung (2B) zumindest einen Wahrnehmungselektrodenpol (200, 201, 203) zum Detektieren von ventrikulären und/oder atrialen Signalen aufweisen.

8. Implantierbares Defibrillationssystem nach Anspruch 7, **dadurch gekennzeichnet, dass** der zumindest eine Wahrnehmungselektrodenpol (200, 201, 203) als Ringelektrode ausgebildet ist.

9. Implantierbares Defibrillationssystem nach einem der Ansprüche 6 bis 8, **dadurch gekennzeichnet, dass** der zumindest eine Defibrillationselektrodenpol (202) als Wendel ausgebildet ist.

10. Implantierbares Defibrillationssystem nach einem der Ansprüche 6 bis 9, **dadurch gekennzeichnet, dass** die erste Elektrodenleitung (2A) und/oder die zweite Elektrodenleitung (2B) zumindest einen atrialen Wahrnehmungselektrodenpol (200, 201) zum Detektieren atrialer Signale an einem dem Defibrillationsgenerator (1) abgewandten, distalen Ende der jeweiligen Elektrodenleitung (2A, 2B) aufweisen.

11. Implantierbares Defibrillationssystem nach einem der Ansprüche 6 bis 10, **dadurch gekennzeichnet, dass** die erste Elektrodenleitung (2A) und/oder die zweite Elektrodenleitung (2B) zwei einen Dipol ausbildende, atriale Wahrnehmungselektrodenpole (200, 201) zum Detektieren atrialer Signale aufweisen.

12. Implantierbares Defibrillationssystem nach einem der Ansprüche 6 bis 11, **dadurch gekennzeichnet, dass** zumindest ein atrialer Wahrnehmungselektrodenpol (200, 201) zum Detektieren atrialer Signale und zumindest ein ventrikulärer Wahrnehmungselektrodenpol (203) zum Detektieren ventrikulärer Signale an unterschiedlichen Seiten des zumindest einen Defibrillationselektrodenpols (202) der jeweiligen Elektrodenleitung (2A, 2B) angeordnet sind.

13. Implantierbares Defibrillationssystem nach einem der Ansprüche 6 bis 12, **gekennzeichnet durch** eine dritte Elektrodenleitung (3) zum Anschließen an einen dritten Anschluss (114) des Anschlussblocks (11) des Defibrillationsgenerators (1), wobei die dritte Elektrodenleitung (3) intravenös im Herz (H) zu implantieren ist.

14. Implantierbares Defibrillationssystem nach Anspruch 13, **dadurch gekennzeichnet, dass** die dritte Elektrodenleitung (3) eine Elektrodenanordnung (30) zur intrakardialen Stimulation und/oder Wahrnehmung aufweist.

15. Verfahren zur Konfiguration eines implantierbaren Defibrillationssystems, aufweisend: Bereitstellen eines Defibrillationsgenerators (1), der ein Gehäuse (10), einen Anschlussblock (11) zum Anschließen einer Elektrodenleitungsbaugruppe (4) und eine Steuereinrichtung (12) zum Steuern des Betriebs des Defibrillationsgenerators (1) aufweist; und Bereitstellen einer zumindest eine Elektrodenleitung (2, 2A, 2B, 3) umfassenden Elektrodenleitungsbaugruppe (4), wobei die Elektrodenleitungsbaugruppe (4) nicht-transvenös in einem Patienten zu implantieren ist; **gekennzeichnet durch:** entweder Anschließen von einer zumindest einen Defibrillationselektrodenpol (202) aufweisenden Elektrodenleitung (2, 2A, 2B) der Elektrodenleitungsbaugruppe (4) an einen von zwei Anschlüssen (110, 112) des Anschlussblocks (11) oder Anschließen von zwei jeweils zumindest einen Defibrillationselektrodenpol (202) aufweisenden Elektrodenleitungen (2, 2A, 2B) der Elektrodenleitungsbaugruppe (4) an die zwei Anschlüsse (110, 112) des Anschlussblocks (11); und Konfigurieren einer Schalteinheit (124) der Steuereinrichtung (12) zum Einnehmen eines ersten Betriebszustands, wenn nur an einen der zwei Anschlüsse (110, 112) eine Elektrodenleitung (2, 2A, 2B) angeschlossen ist, oder eines zweiten Betriebszustands, wenn an die zwei Anschlüsse (110, 112) jeweils eine Elektrodenleitung (2, 2A, 2B) angeschlossen ist, wobei die Steuereinrichtung (12) in dem ersten Betriebszustand eine Defibrillationsfunktion über die an den einen der Anschlüsse (110, 112) angeschlossene Elektrodenleitung (2, 2A, 2B) und in dem zweiten Betriebszustand eine Defibrillationsfunktion über die an die zwei Anschlüsse (110, 112) angeschlossenen Elektrodenleitungen (2, 2A, 2B) bereitstellt.
